# EUROPEAN PATENT APPLICATION

(11) **EP 2 952 187 A1**
(43) Date of publication of application: **09.12.2015**
(21) Application number: 14746787.2
(22) Date of filing: 30.01.2014
(51) Int. Cl.: A61K 31/4184, A61K 9/20, A61K 47/10, A61K 47/34, A61K 47/38, A61P 9/12, A61P 43/00

(54) **PHARMACEUTICAL COMPOSITION CONTAINING CANDESARTAN CILEXETIL**

(30) Priority: 30.01.2013 JP 2013015099
(71) Applicant: Sawai Pharmaceutical Co., Ltd., Yodogawa-ku Osaka-shi Osaka 532-0003 (JP)
(72) Inventor: FUKUHARA, Yasushi, Osaka City Osaka 532-0003 (JP); UETSUKI, Kenji, Osaka City Osaka 532-0003 (JP); NAKAGAWA, Tomoya, Osaka City Osaka 532-0003 (JP)
(74) Representative: Von Kreisler Selting Werner - Partnerschaft von Patentanwälten und Rechtsanwälten mbB
(86) International application number: PCT/JP2014/052126
(87) International publication number: WO 2014/119667

(57) **Abstract**

The present invention provides a pharmaceutical composition containing candesartan cilexetil, which is produced by wet granulation using a dispersion liquid of candesartan cilexetil together with a powdery additive. The dispersion liquid may also contain a water-soluble polymer, a sugar alcohol, or a stabilizer, and the water-soluble polymer may be hydroxypropylcellulose. The sugar alcohol may be mannitol. In addition, the stabilizer may be lauromacrogol.

## Description

### TECHNICAL FIELD

The present invention relates to a pharmaceutical composition containing candesartan cilexetil. The invention particularly relates to a pharmaceutical composition containing candesartan cilexetil in which a dissolution failure of candesartan cilexetil is improved.

### BACKGROUND ART

Candesartan cilexetil, that is an angiotensin II receptor antagonist, has been widely used as a therapeutic drug for hypertension.

Candesartan cilexetil is poorly soluble in water. Therefore, the dissolution thereof from a solid preparation needs to be improved in order that candesartan cilexetil is satisfactorily absorbed by the body. For example, a method of using fine particle of candesartan cilexetil to improve the dissolution thereof from a solid preparation is known. However, it is known that the crystallinity of candesartan cilexetil is reduced by pressure or the like during the pulverizing of candesartan cilexetil, leading to a decrease in the purity of candesartan cilexetil. Then, Patent Literature 1 proposes a method for the preparation of the stable candesartan cilexetil of fine particle size, in which a slurry of candesartan cilexetil having a fine particle size is held under a warming condition for a predetermined period of time, then filtered, and the residue of filtering is dried. In addition, Patent Literature 2 proposes a wet pulverization method for the preparation of micronized candesartan cilexetil, in which candesartan cilexetil is dissolved or suspended in water together with a binder, etc. However, in both methods, the operations during production are complicated.

In addition, Patent Literature 3 proposes a method for the preparation of the amorphous of candesartan cilexetil, in which candesartan cilexetil is dispersed in a matrix containing a solubilizer or a water-soluble polymer to improve the absorbability thereof in the body. However, the operations during production, including melting/solidification, milling, etc., are extremely complicated.

Further, Patent Literatures 4 to 6 propose a method in which a surfactant and the like are added in a solid preparation containing candesartan cilexetil to improve the dissolution of candesartan cilexetil from the preparation, etc. However, it is hard to say that these methods have achieved improvement to a practically desired level.

### CITATION LIST

### PATENT LITERATURE

Patent Literature 1: Published Japanese Translation of PCT Patent Application No. 2008-505935
Patent Literature 2: Japanese Patent Application Laid-Open No. 2012-153631
Patent Literature 3: Published Japanese Translation of PCT Patent Application No. 2010-502698
Patent Literature 4: Published Japanese Translation of PCT Patent Application No. 2008-536929
Patent Literature 5: Published Japanese Translation of PCT Patent Application No. 2010-522692
Patent Literature 6: Published Japanese Translation of PCT Patent Application No. 2010-535212

### SUMMARY OF INVENTION

### TECHNICAL PROBLEM

As mentioned above, although a large number of methods for improving the solubility of candesartan cilexetil have been reported, they have problems in that the production process is complicated, etc. Thus, these methods have hardly been practically sufficient.

The present invention is aimed at solving the problems mentioned above. An object thereof is to provide to a pharmaceutical composition containing candesartan cilexetil in which a dissolution failure of candesartan cilexetil is improved.

### SOLUTION TO PROBLEMS

According to one embodiment of the present invention, a pharmaceutical composition containing candesartan cilexetil, which is produced by wet granulation using a dispersion liquid of candesartan cilexetil dispersed in a solvent together with a powdery additive, is provided.

The dispersion liquid used in the production process of the pharmaceutical composition containing candesartan cilexetil may also contain a water-soluble polymer.

The water-soluble polymer in the dispersion liquid used in the production process of the pharmaceutical composition containing candesartan cilexetil may be hydroxypropylcellulose.

The dispersion liquid used in the production process of the pharmaceutical composition containing candesartan cilexetil may further contain a sugar alcohol.

The sugar alcohol in the dispersion liquid used in the production process of the pharmaceutical composition containing candesartan cilexetil may be mannitol.

The dispersion liquid used in the production process of the pharmaceutical composition containing candesartan cilexetil may further contain a stabilizer.

The stabilizer in the dispersion liquid used in the production process of the pharmaceutical composition containing candesartan cilexetil may be lauromacrogol.

### ADVANTAGEOUS EFFECTS OF INVENTION

The present invention provides a pharmaceutical composition containing candesartan cilexetil in which a dissolution failure of candesartan cilexetil is improved.

### BRIEF DESCRIPTION OF DRAWINGS

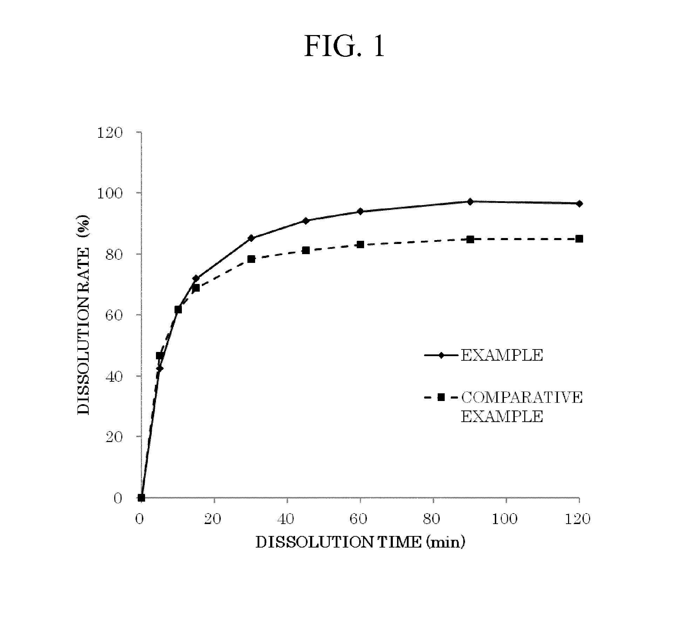

Figure 1 shows a result of the dissolution of candesartan cilexetil according to one embodiment of the present invention.

### DESCRIPTION OF EMBODIMENTS

The present inventors have found that by means of the wet granulation method, which is performed using a dispersion liquid of candesartan cilexetil dispersed in a solvent together with a powdery additive, the dissolution failure can be improved. They have thus accomplished the present invention.

In a conventional art, a method in which powdery candesartan cilexetil is granulated together with other additives has been common. In the present invention, candesartan cilexetil does not need to be finely pulverized in advance. The present invention provides a wet granulation method which is performed using a dispersion liquid of candesartan cilexetil dispersed in a solvent together with a powdery additive, which is a simple method that has not been heretofore reported.

Hereinafter, the pharmaceutical composition containing candesartan cilexetil according to the present invention and a method for producing the same will be described. However, the pharmaceutical composition containing candesartan cilexetil of the present invention and a method for producing the same are not limited to the description in the following embodiments and examples.

Candesartan cilexetil is an active ingredient of the pharmaceutical composition containing candesartan cilexetil according to the present invention, and its chemical name is (RS)-1-[(cyclohexyloxy)carbonyloxy]ethyl 2-ethoxy-1-{[2'-(1H-tetrazole-5-yl)-biphenyl-4-yl]methyl}-1H-benzimidazole-7-carboxylate.

In the present invention, wet granulation is performed using a dispersion liquid of candesartan cilexetil dispersed in a pharmacologically acceptable solvent together with a powdery additive. A preferred pharmacologically acceptable solvent for dispersing candesartan cilexetil is water, and it is possible to use purified water.

In one embodiment, it is preferable that the dispersion liquid of candesartan cilexetil contains a water-soluble polymer. The water-soluble polymer can improve the dispersibility of candesartan cilexetil in the solvent.

Examples of usable water-soluble polymers according to this embodiment include hydroxypropylcellulose, methylcellulose, hydroxypropylmethylcellulose, povidone, and polyvinyl alcohol, or the like. In this embodiment, it is preferable to use hydroxypropylcellulose (HPC) as a water-soluble polymer. In this embodiment, it is preferable that the amount of water-soluble polymer is within a range of 0.01 parts by weight or more and 3 parts by weight or less, more preferably 0.05 parts by weight or more and 0.5 parts by weight or less, based on 100 parts by weight of the pharmaceutical composition containing candesartan cilexetil.

In one embodiment, the dispersion liquid of candesartan cilexetil may further contain a sugar alcohol. In this embodiment, examples of usable sugar alcohols include mannitol, erythritol, xylitol, sorbitol, trehalose, maltitol, or the like. In this embodiment, it is preferable to use mannitol as a sugar alcohol. In this embodiment, it is preferable that the amount of sugar alcohol is within a range of 0.5 parts by weight or more and 50 parts by weight or less, more preferably within a range of 5 parts by weight or more and 20 parts by weight or less, based on 100 parts by weight of the pharmaceutical composition containing candesartan cilexetil.

In one embodiment, the dispersion liquid of candesartan cilexetil may further contain a stabilizer. In this embodiment, examples of usable stabilizers include higher alcohols such as stearyl alcohol; fatty acid esters of polyalcohols, such as sucrose fatty acid ester and sorbitan fatty acid ester; polymers or copolymers of alkylene oxides, such as polyethylene glycol; and higher alcohol ethers of polyalcohols, such as lauromacrogol. Preferred examples of lauromacrogol include polyoxyethylene (2) lauryl ether, polyoxyethylene (4.2) lauryl ether, polyoxyethylene (9) lauryl ether, polyoxyethylene (21) lauryl ether, and polyoxyethylene (25) lauryl ether. In particular, solid polyoxyethylene (21) lauryl ether and polyoxyethylene (25) lauryl ether are more preferable for use in the pharmaceutical composition containing candesartan cilexetil according to the present invention. In this embodiment, it is preferable that the amount of stabilizer contained is 0.01 parts by weight or more and 2.4 parts by weight or less based on 100 parts by weight of the pharmaceutical composition containing candesartan cilexetil.

The pharmaceutical composition containing candesartan cilexetil according to the present invention may contain at least one pharmacologically acceptable additive selected from commonly used diluents, disintegrants, binders, and stabilizers.

Examples of diluents include crystalline cellulose, starches such as corn starch, saccharides such as sucrose, lactose, and glucose, sugar alcohols such as mannitol, erythritol, xylitol, and sorbitol, light anhydrous silicic acid, talc, magnesium oxide, magnesium carbonate, calcium carbonate, anhydrous dibasic calcium phosphate, and tribasic calcium phosphate. These fillers may be used alone, and it is also possible to use a combination of two or more kinds.

Examples of disintegrants include crystalline cellulose, sodium carboxymethyl starch, carmellose, carmellose calcium, carmellose sodium, croscarmellose sodium, crosslinked polyvinyl pyrrolidone, low-substituted hydroxypropylcellulose, crospovidone, and starches. These disintegrants may be used alone, and it is also possible to use a combination of two or more kinds.

Examples of binders include pharmacologically acceptable binders, such as sucrose, gelatin, powdered acacia, methylcellulose, hydroxypropylmethylcellulose, hydroxypropylcellulose, carboxymethylcellulose (carmellose), crystalline cellulose-sodium carboxymethylcellulose, polyvinyl pyrrolidone, pullulan, dextrin, tragacanth, sodium alginate, pregelatinized starch, and polyvinyl alcohol. These binders may be used alone, and it is also possible to use a combination of two or more kinds.

Examples of stabilizers include higher alcohols such as stearyl alcohol; fatty acid esters of polyalcohols, such as sucrose fatty acid ester and sorbitan fatty acid ester; polymers or copolymers of alkylene oxides, such as polyethylene glycol; and higher alcohol ethers of polyalcohols, such as lauromacrogol. These stabilizers may be used alone, and it is also possible to use a combination of two or more kinds.

In addition to the pharmacologically acceptable additives such as diluents, disintegrants, binders, and stabilizers, the pharmaceutical composition containing candesartan cilexetil may further contain pharmacologically acceptable, commonly used other additives such as lubricants, coating agents, preservatives, corrigents, sweeteners, colorants, flavoring agents, fragrances, fluidizers, and polishing agents.

Examples of lubricants include magnesium stearate, light anhydrous silicic acid, talc, calcium stearate, sodium stearyl fumarate, sucrose fatty acid ester, and L-leucine. Examples of sweeteners include saccharides such as sucrose, lactose, and glucose, sugar alcohols such as mannitol, erythritol, xylitol, and sorbitol, aspartame, sucralose, acesulfame potassium, and thaumatin. These commonly used other additives may be used alone, and it is also possible to use a combination of two or more kinds.

### (Production Method)

As mentioned above, the pharmaceutical composition containing candesartan cilexetil according to the present invention can be produced by wet granulation using a dispersion liquid of candesartan cilexetil dispersed in a solvent together with a powdery additive.

Of the production process of the pharmaceutical composition containing candesartan cilexetil according to this embodiment, the step of preparing a dispersion liquid of candesartan cilexetil will be described. First, a water-soluble polymer is added to a solvent and dissolved using a mixer. At this time, in addition to the water-soluble polymer, a sugar alcohol and a stabilizer may also be added. Subsequently, candesartan cilexetil is added to the solution and dispersed using a disperser.

The production process of the pharmaceutical composition containing candesartan cilexetil according to this embodiment will be described. Granulation is performed by a wet process using the candesartan cilexetil dispersion liquid together with a powdery additive, and the obtained granulated product is subjected to drying, particle size regulation, and tableting in the usual manner. The powdery additive herein is at least one pharmacologically acceptable additive selected from diluents, disintegrants, binders, and stabilizers mentioned above. In addition, after particle size regulation and before tableting, a diluent, a disintegrant, a lubricant, or the like may further be mixed.

In this embodiment, the granulation operation may be performed using apparatus such as, for example, a fluidized-bed granulator, an agitation granulator, a biaxial granulator, or the like. In this embodiment, it is preferable to use a fluidized-bed granulation method, where wet granulation using a candesartan cilexetil dispersion liquid and drying can be performed continuously.

Tableting may be performed using a commercially available tableting machine.

In this embodiment, an uncoated tablet or a post-granulation uncoated granule may be coated. In that case, it is preferable that coating is performed by means of a film coating machine, a fluidized-bed granulator, or the like. Coating can be performed with a coating agents well known in the field such as, for example, hydroxypropylmethylcellulose, hydroxypropylcellulose, methylcellulose, polyvinyl alcohol, polyvinylpyrrolidone, polyethylene glycol, hydroxypropylmethylcellulose acetate succinate, hypromellose phthalate, cellulose acetate phthalate, aminoalkyl methacrylate copolymer E, aminoalkyl methacrylate copolymer RS, methacrylic acid copolymer L, methacrylic acid copolymer LD, methacrylic acid copolymer S, dried methacrylic acid copolymer LD, ethyl acrylate-methyl methacrylate copolymer dispersion, sucrose, and the like.

According to the method for producing a pharmaceutical composition containing candesartan cilexetil according to this embodiment, the wet granulation is performed using a dispersion liquid of candesartan cilexetil dispersed in a solvent together with a powdery additive. As a result, the dissolution of candesartan cilexetil from a pharmaceutical composition containing candesartan cilexetil can be improved.

### EXAMPLES

The pharmaceutical composition containing candesartan cilexetil according to the present invention mentioned above will be described in further detail through specific production examples and test results thereof.

### (Example)

65 g of D-mannitol, 0.75 g of hydroxypropylcellulose (HPC), and 0.5 g of lauromacrogol were added to 848.75 g of purified water and dissolved by using a mixer (manufactured by PRIMIX Corporation) at 2000 rpm for 20 minutes. Subsequently, the solution was transferred to a disperser (manufactured by PRIMIX Corporation), and 60 g of candesartan cilexetil was added thereto and stirred at 8000 rpm for 20 minutes to disperse candesartan cilexetil in the solution.

367.25 g of D-mannitol, 70 g of low-substituted hydroxypropylcellulose (L-HPC), 2 g of powder hydrogenated maltose starch syrup, and 3 g of sucralose were put through a No. 30 sieve and placed in a fluidized-bed granulator (manufactured by Powrex Corp.). And fluidized-bed granulation was performed by adding the obtained candesartan cilexetil dispersion liquid thereto. The obtained granulated product was dried and the particle size of the dried product was regulated through a No. 22 sieve. The obtained particle-size-regulated product, 40 g of stearic acid, 32.5 g of crystalline cellulose, 6.5 g of magnesium aluminometasilicate (Neusilin UFL2), and 2.5 g of magnesium stearate were mixed by a V-blender (manufactured by Fuji Paudal Co., Ltd.), and the obtained mixture was tableted by a rotary tableting machine (manufactured by Kikusui Seisakusho Ltd.) such that the tablets would each have a weight of 130.0 mg and a thickness of 3.30 mm, thereby the tablets were obtained.

### (Comparative Example)

65 g of D-mannitol, 0.75 g of hydroxypropylcellulose (HPC), and 0.5 g of lauromacrogol were added to 908.75 g of purified water and dissolved by using a mixer (manufactured by PRIMIX Corporation) at 2000 rpm for 20 minutes. 60 g of candesartan cilexetil, 367.25 g of D-mannitol, 70 g of low-substituted hydroxypropylcellulose (L-HPC) , 2 g of powder hydrogenated maltose starch syrup, and 3 g of sucralose were put through a No. 30 sieve and placed in a fluidized-bed granulator (manufactured by Powrex Corp.). And fluidized-bed granulation was performed by adding the obtained solution thereto. The obtained granulated product was dried and the particle size of the dried product was regulated through a No. 22 sieve. The obtained particle-size-regulated product, 40 g of stearic acid, 32.5 g of crystalline cellulose, 6.5 g of magnesium aluminometasilicate (Neusilin UFL2), and 2.5 g of magnesium stearate were mixed by a V-blender (manufactured by Fuji Paudal Co., Ltd.), and the obtained mixture was tableted by a rotary tableting machine (manufactured by Kikusui Seisakusho Ltd.) such that the tablets would each have a weight of 130.0 mg and a thickness of 3.30 mm, thereby the tablets were obtained.

When the pharmaceutical compositions containing candesartan cilexetil of the example and the comparative example were tableted by a tableting machine as mentioned above, however, severe sticking was observed in the comparative example, while sticking was not observed in the example.

### (Dissolution)

With respect to the example and the comparative example, the dissolution of candesartan cilexetil was examined. The dissolution test was performed in accordance with the Guideline for Bioequivalence Studies of Generic Products. The dissolution rate (%) of candesartan cilexetil was measured for up to 120 minutes under the condition of 50 rpm using a 0.1% Tween pH 6.8 solution. Fig. 1 shows the results of the measurement of candesartan cilexetil dissolution in the example and the comparative example. As is obvious from Fig. 1, in the example, improved dissolution was observed as compared with that in the comparative example.

## Claims

1. A pharmaceutical composition containing candesartan cilexetil, characterized that the pharmaceutical composition is produced by wet granulation using a dispersion liquid of candesartan cilexetil together with a powdery additive.

2. The pharmaceutical composition containing candesartan cilexetil according to claim 1, **characterized in that** the dispersion liquid contains a water-soluble polymer.

3. The pharmaceutical composition containing candesartan cilexetil according to claim 2, **characterized in that** the water-soluble polymer is hydroxypropylcellulose.

4. The pharmaceutical composition containing candesartan cilexetil according to claim 2, **characterized in that** the dispersion liquid further contains a sugar alcohol.

5. The pharmaceutical composition containing candesartan cilexetil according to claim 4, **characterized in that** the sugar alcohol is mannitol.

6. The pharmaceutical composition containing candesartan cilexetil according to claim 2, **characterized in that** the dispersion liquid further contains a stabilizer.

7. The pharmaceutical composition containing candesartan cilexetil according to claim 6, **characterized in that** the stabilizer is lauromacrogol.
